# EUROPEAN PATENT SPECIFICATION

(11) **EP 2 501 417 B1**
(45) Date of publication and mention of the grant of the patent: **31.08.2016**
(21) Application number: 10832085.4
(22) Date of filing: 17.11.2010
(51) Int. Cl.: A61L 12/12, A01N 59/00

(54) **A HYDROGEN PEROXIDE SOLUTION AND KIT FOR DISINFECTING CONTACT LENSES**
WASSERSTOFFPEROXIDLÖSUNG UND KIT ZUR DESINFEKTION VON KONTAKTLINSEN
SOLUTION DE PEROXYDE D'HYDROGÈNE ET KIT POUR LA DÉSINFECTION DE LENTILLES DE CONTACT

(30) Priority: 17.11.2009 US 261844 P; 19.11.2009 US 262674 P
(43) Date of publication of application: 26.09.2012
(73) Proprietor: Novartis AG, 4056 Basel (CH)
(72) Inventor: MINICK, Kasey, Jon, Cumming GA 30041 (US); GABRIEL, Manal, M., Marietta GA 30062 (US); MUYA, Leroy, Wainaina, Duluth GA 30096 (US); NASH, Walter, Lee, Duluth GA 30096 (US); MINNO, George, Edward, Windham NH 03087 (US)
(74) Representative: Bohest AG
(86) International application number: PCT/US2010/056981
(87) International publication number: WO 2011/062959

(56) References cited:
- WO-A1-94/14479
- US-A- 5 130 124
- US-A- 5 468 448
- US-A- 5 523 012
- US-A- 6 148 992
- US-A1- 2008 314 767
- KRISTINE DALTON ET AL: "Physical Properties of Soft Contact Lens Solutions", OPTOMETRY AND VISION SCIENCE, vol. 85, no. 2, 28 February 2002 (2002-02-28), pages 122-128, XP055063513, DOI: 10.1097/OPX.0b013e318162261e
- PANARIN E F ET AL: "Complexation of hydrogen peroxide with polyvinylpyrrolidone: ab initio calculations", EUROPEAN POLYMER JOURNAL, PERGAMON PRESS LTD. OXFORD, GB, vol. 37, no. 2, 1 February 2001 (2001-02-01), pages 375-379, XP004219652, ISSN: 0014-3057, DOI: 10.1016/S0014-3057(00)00154-3

## Description

### BACKGROUND OF THE INVENTION

### FIELD OF THE INVENTION

This invention relates generally to disinfection and cleaning systems for medical devices. In a preferred embodiment, the invention relates to compositions, methods and articles for simultaneously cleaning and disinfecting contact lenses.

### DESCRIPTION OF THE RELATED ART

Disinfecting solutions for use with contact lenses are well known in the art and the use of such lenses involves a daily disinfecting treatment. Flexible, or soft, contact lenses are generally made from hydrophilic polymers and the hydroxy groups of these lenses attract and retain substantial amounts of water in the plastic which results in difficulties during cleaning and sterilization.

The two most common methods of contact lens disinfection are the multipurpose solutions in which a single solution is used for disinfecting, cleaning, and storing lenses and hydrogen peroxide-based systems. The multipurpose solutions contain preservative but hydrogen peroxide-based systems contain no preservative. Hydrogen peroxide is an effective microbial disinfectant, destroying pathogens by oxidation. Hydrogen peroxide systems, particularly 3% hydrogen peroxide solutions, emerged as the disinfectant of choice for all types of daily and extended wear hydrogel lenses. The primary reason for its popularity is its rapid kill of microbial contaminants and its low-residual character. After hydrogen peroxide disinfects lenses, it can be converted into innocuous and natural by-products, such as O₂ and water, which are compatible with ocular physiology. See Krezanoski et al., "Journal of the American Optometric Association", Vol. 59, Number 3, pages 193 197 (1988).

In general, the hydrogen peroxide systems involve a hydrogen peroxide-containing disinfecting solution into which the contact lenses to be disinfected are placed and allowed to remain for a required period of time. The hydrogen peroxide may (1) oxidize chloride in the bacteria to hypochlorite or (2) decompose into nascent oxygen and hydroxyl radicals, thus providing a germicidal effect. Following the requisite time period a purposeful inactivation of the hydrogen peroxide is conducted, for example, with a platinum catalyst. Following inactivation, the contact lens may be reinserted into the eye.

A great deal of patent literature is available concerning hydrogen peroxide contact lens disinfection systems. Reference is made in this respect to the following:
U.S. Pat. No. 5,523,012 to Winterton, et al. teaches that the addition of a surface-active agent to a peroxide disinfection solution will enhance the disinfecting properties of the solution. However, the surfactants disclosed are all present in amounts above 0.1 % and, because of excessive foaming, are incompatible with the platinum catalyst disc typically used to deactivate hydrogen peroxide in the lens disinfection systems.
U.S. Pat. No. 5,746,972 to Park, et al. teaches compositions and methods for disinfecting and cleaning contact lenses include a liquid medium containing hydrogen peroxide and a solid ethylene oxide/propylene oxide block copolymer surfactant having at least 70% by weight polyethylene oxide. The hydrogen peroxide is degraded by a catalase released into the solution and causes "a reduced amount of foam." However, such compositions cause excessive foaming when a platinum catalyst is used to decompose the hydrogen peroxide.
WO 94/14479 (Bausch & Lomb) discloses to use hydrogen peroxide as a PVP-hydrogen peroxide complex in solid form in order to stabilize hydrogen peroxide in solid form before it is applied into a solution.

One long felt need in the contact lens industry is to provide a convenient yet high disinfecting efficacy contact lens care solution. There are two type of hydrogen peroxide contact lens disinfection solution and they are: one-step system and two- step system. The two- step system involves:
first, soaking the contact lens in a hydrogen peroxide solution having a concentration of between 0.6% to 3% for a period of time, during the period of time the hydrogen peroxide concentration is almost constant and
second, soaking the contact lens in a catalytic solution to neutralization of the hydrogen peroxide left in contact lens. The one-step system involves soaking contact lens in about 3% hydrogen peroxide solution for about 6 hours in a contact lens storage case in which a catalytic platinum disk is located. After 6 hours soaking, the concentration of residual hydrogen peroxide is reduced to less than 200 ppm, more preferred 100 ppm and the contact lens may be inserted to eye. Typically, the two- step system has a higher disinfecting efficacy than the one step system because the former has a higher hydrogen peroxide concentration during the disinfecting treatment. However, the one-step system is more convenient than the two-step system and has gained a much higher popularity among contact lens wearer.

Therefore, it would be advantageous to provide peroxide contact lens disinfection solutions that overcomes one or more of these problems.

### SUMMARY OF THE INVENTION

The present invention, in one aspect, provides a contact lens care solution for disinfecting a contact lens comprising an effective disinfecting amount of hydrogen peroxide of 2% to 6% by weight and a copolymer of vinylpyrrolidone, wherein the copolymer of vinylpyrrolidone is present in an amount of 0.1 % to 5% by weight per volume (w/v) sufficient to provide an increased residual hydrogen peroxide concentration at 30 minutes treatment by at least 20% relative to a control solution having identical composition without the copolymer of vinylpyrrolidone in the presence of an identical catalyst, one or more buffering agents in an amount sufficient to provide the composition a pH of from 6.0 to 8.0, wherein the composition has an osmolality of from 200 to 450 mOsm/kg and a viscosity up to 5.0 centipoises at 25°C, wherein the copolymer is a copolymer of vinylpyrrolidone and at least one amino-containing vinylic monomer, wherein the amino-containing vinylic monomer is selected from the group consisting of alkylaminoalkylmethacrylate having 8-15 carbon atoms, alkylaminoalkylacrylate having 7-15 carbon atoms, dialkylaminoalkyl-methacrylate having 8-20 carbon atoms, dialkylaminoalkylacrylate having 7-20 carbon atoms, and N-vinylalkylamide having 3-10 carbon atoms.

The present invention, in another aspect, provides a kit for use in the disinfection of contact lenses comprising: a) an aqueous hydrogen peroxide solution as disclosed in the preceding paragraph, and b) a container for holding a given amount of hydrogen peroxide solution, wherein the container contains a disc wherein the disc comprises a substrate and a catalyst coating thereon, wherein, when the given amount of hydrogen peroxide is added to the container to entirely submerge the catalyst coated radial disc, the aqueous hydrogen peroxide solution decomposes over a period time of six hours to provide a residual hydrogen peroxide concentration of less than 100 ppm.

These and other aspects of the invention will become apparent from the following description of the presently preferred embodiments. The detailed description is merely illustrative of the invention and does not limit the scope of the invention, which is defined by the appended claims and equivalents thereof. As would be obvious to one skilled in the art, many variations and modifications of the invention may be affected without departing from the spirit and scope of the novel concepts of the disclosure.

### Brief Description of the Drawings

FIGURE 1 is a top view of a prior art trigon catalyst disc.
FIGURE 2 is a top view of a radial catalyst disc according to an example embodiment of the present invention.
FIGURE 3 is a top view of a radial catalyst disc with 2 arm shaved off according to an example embodiment of the present invention.
FIGURE 4 is a top view of a radial catalyst disc with 4 arm shaved off according to an example embodiment of the present invention.
FIGURE 5 is a top view of a shaved catalyst radial disc according to an example embodiment of the present invention.
FIGURE 6 is a perspective view of a radial catalyst disc according to an example embodiment of the present invention.
FIGURE 7 is a perspective view of a 50% height modified radial catalyst disc according to an example embodiment of the present invention.
FIGURE 8A-E is a bar chart illustrating residual hydrogen peroxide concentrations after cycling surface area modified radial catalyst discs in Clear Care™ for 20, 30, 60, 120 and 360 minutes.

### DETAILED DESCRIPTION OF THE INVENTION

Unless defined otherwise, all technical and scientific terms used herein have the same meaning as commonly understood by one of ordinary skill in the art to which this invention belongs. Generally, the nomenclature used herein and the laboratory procedures are well known and commonly employed in the art. Conventional methods are used for these procedures, such as those provided in the art and various general references. Where a term is provided in the singular, the inventors also contemplate the plural of that term. The nomenclature used herein and the laboratory procedures described below are those well known and commonly employed in the art. As employed throughout the disclosure, the following terms, unless otherwise indicated, shall be understood to have the following meanings.

The present invention is based on the discovery that the disinfecting efficacy against a wide spectrum of microorganisms can be improved by (A) incorporating a copolymer of vinylpyrrolidone as defined in claim 1 to hydrogen peroxide solution or (B) by reducing the surface area of catalyst disc which is used to neutralize the hydrogen peroxide in combination with (A).

A disinfecting solution is generally defined as a contact lens care product containing one or more active ingredients (for example, anti-microbial agents and/or preservatives) in sufficient concentrations to destroy harmful microorganisms on the surface of a contact lens within the recommended minimum soaking time. The recommended minimum soaking time is included in the package instructions for use of the disinfecting solution.

The term "soft lens" means a lens having a proportion of hydrophilic repeat units such that the water content of the lens during use is at least 20% by weight. The term "soft contact lens" as used herein generally refers to those contact lenses which readily flex under small amounts of force. Typically, soft contact lenses are formulated from polymers having a certain proportion of repeat units derived from hydroxyethyl methacrylate and/or other hydrophilic monomers, typically crosslinked with a crosslinking agent. In contrast, conventional "hard contact lenses," which cover only a part of the cornea of the eye, usually consist of poly (methyl methacrylate) crosslinked with ethylene glycol dimethacrylate or the like, and conventional rigid gas permeable lenses (RGP) typically consists of monomers containing silicon that result in a more oxygen-permeable material.

By the term "ophthalmically safe" with respect to a contact-lens solution is meant that a contact lens treated with the solution is safe for direct placement on the eye without rinsing, that is, the solution is safe and sufficiently comfortable for daily contact with the eye via a contact lens. An ophthalmically safe solution has a tonicity and pH that is compatible with the eye and comprises materials, and amounts thereof, that are non-cytotoxic according to international ISO standards and U.S. FDA regulations.

The term "compatible with the eye" means a solution that may be in intimate contact with the eye for an extended period of time without significantly damaging the eye and without significant user discomfort.

The term "disinfecting solution" means a solution containing one or more microbiocidal compounds that is effective for reducing or substantially eliminating the presence of an array of microorganisms present on a contact lens, which can be tested by challenging a solution or a contact lens after immersion in the solution with specified inoculums of such microorganisms. The term "disinfecting solution" as used herein does not exclude the possibility that the solution may also be useful for a storage solution or that the disinfecting solution may additionally be useful for daily cleaning, rinsing, and storage of contact lenses.

The term "cleaning" means that the solution contains one or more active ingredients in sufficient concentrations to loosen and remove loosely held lens deposits and other contaminants on the surface of the article. While not necessary with the present invention, a user may wish to use the solutions of the present invention in conjunction with digital manipulation (for example, manual rubbing of the lens with a solution) or with an accessory device that agitates the solution in contact with the lens, for example, a mechanical cleaning aid.

A solution that is useful for cleaning, chemical disinfection, storing, and rinsing an article, such as a contact lens, is referred to herein as a "multi-purpose solution." Such solutions may be part of a "multi-purpose solution system" or "multi-purpose solution package." The procedure for using a multi-purpose solution, system or package is referred to as a "multi-functional disinfection regimen." Multi-purpose solutions do not exclude the possibility that some wearers, for example, wearers particularly sensitive to chemical disinfectants or other chemical agents, may prefer to rinse or wet a contact lens with a another solution, for example, a sterile saline solution prior to insertion of the lens. The term "multi-purpose solution" also does not exclude the possibility of periodic cleaners not used on a daily basis or supplemental cleaners for removing proteins, for example enzyme cleaners, which are typically used on a weekly basis.

"Molecular weight" of a polymeric material, as used herein, refers to the number-average molecular weight unless otherwise specifically noted or unless testing conditions indicate otherwise.

"An increased residual hydrogen peroxide concentration relative to a control solution", as used herein, refers to the increased residual hydrogen peroxide concentration relative to a control solution having identical composition without the copolymer of vinylpyrrolidone as defined herein in the presence of an identical catalyst. The percentage of the increased residual hydrogen peroxide concentration due to the presence of copolymer of vinylpyrrolidone is defined as ratio of [(C_{with at 30 minute} - C_{control} at ₃₀ minute) /C_{control} at _{30 minute} )] X 100; C_{with at 30 minute} is residual hydrogen peroxide concentration measured when a fixed volume of hydrogen peroxide containing solution having the copolymer of vinylpyrrolidone contacts with a suitable catalyst in a plastic container for 30 minutes at room temperature. C_{control at 30 minute} is residual hydrogen peroxide concentration measured when a fixed volume of the identical hydrogen peroxide solution except of having no copolymer of vinylpyrrolidone contacts ( called control solution) with the identical suitable catalyst in the identical plastic container for 30 minutes at room temperature. The fixed volume is capable to totally immerse the catalyst and is from 5 cm³ to 25 cm³, preferred from 7 cm³ to 18 cm³, more preferred from 8 cm³ to 13 cm^{3,} even more preferred from 9 cm³ to 11 cm³. Hydrogen peroxide solution comprises hydrogen peroxide, and may include other suitable ingredients for contact lens care solution, for example, surfactant, buffer, tonicity agent.

The term "an increased residual hydrogen peroxide concentration relative to a control disc ", as used herein, refers to the increased residual hydrogen peroxide concentration relative to a control disc having a substrate surface of 10.4 cm² and a substantially identical catalytic coating weight per surface area when in contact with a fixed volume of the identical hydrogen peroxide solution in the container, wherein the radial disc is positioned in the container to be in contact with the aqueous hydrogen peroxide solution. The percentage of the increased residual hydrogen peroxide concentration due to the less surface area of a disc than the control radial disc is defined as ratio of [(C _{reduced surface area at 60 minute} - C_{control disc at 60 minute}) / C_{control} at _{60 minute} )] X 100; C reduced surface area at ₆₀ minute is residual hydrogen peroxide concentration measured when a fixed volume of hydrogen peroxide contacts with a catalyst coated disc having a less surface area than the control disc in a plastic container for 60 minutes at room temperature. C_{control at 60 minute} is residual hydrogen peroxide concentration measured when a fixed volume of the identical hydrogen peroxide solution contact with a control disc coated with the identical catalyst in the identical plastic container for 60 minutes at room temperature. The fixed volume is capable to totally immerse the catalyst and is from 5 cm³ to 25 cm³, preferred from 7 cm³ to 18 cm³, more preferred from 8 cm³ to 13 cm^{3,} even more preferred from 9 cm³ to 11 cm³. Hydrogen peroxide solution comprises hydrogen peroxide, and may include other suitable ingredients for contact lens care solution, for example, surfactant, buffer, tonicity agent.

According to the present invention, the term "substrate" means that any plastic material. Preferred the plastic material has material characteristics of good dimensional stability, hydrolytically stable and low to no water absorption, for examples, Acetal (Delrin®, Celcon®), CPVC, Engage®, Halar®, Isoplast™Kynar®(PVDF), Lexan®(PC), Noryl® (PPE), Polystyrene (PS), Polypropylene (PP). More preferred the plastic material is Noryl®, modified polyphenylene ether. The disc of the present invention can be made by injection molding process using the plastic material. The disc of the present invention can have a smooth surface and rough surface. Preferred the disc surface has SPI (Society of the Plastics Industry) surface finishes rating from B-2 to C-1, more preferred with B3 rating.

According with the invention, the term "catalysts" means that any material that catalyses the decomposition of hydrogen peroxide. The catalyst is preferably a solid, and more preferably a metal or metal oxide of transition metal from Periods 3 to 12 of the Periodic Table, or one of the lanthanide elements. Particularly preferred is platinum, more particularly platinum oxide. Different transition metals follow different reaction pathways in the decomposition of hydrogen peroxide. For example, applicants believe the peroxide is decomposed using platinum ion as catalyst following the following mechanism:

2H₂ O₂ + Ptⁿ⁺ →2H₂O + O₂ + Ptⁿ⁺

Applicants also discover that the peroxide can be decomposed using Fenton reagents, for example, ferrous ion Fe²⁺ is oxidized by hydrogen to ferric iron Fe³⁺' a hydroxyl radical and a hydroxyl anion. Ferric iron Fe³⁺ is then reduced back to ferrous ion, peroxide radical and a proton by the same hydrogen peroxide (disproportionation).

H₂ O₂ + Fe²⁺ → HO▪ +H O⁻ + Fe³⁺

H₂ O₂ + Fe³⁺→ Fe²⁺ + OOH▪ + H⁺

The highly reactive species the hydroxyl radicals can increase the effectiveness of the disinfection. The Fenton reagent could either be incorporated along side the platinum or alone to act as the catalyst for decomposition of hydrogen peroxide. The sources of Fenton reagents could be, for example, TiO₂, Fe (NO₃)₂, Fe (Cl₂), Fe (NH₄) (SO₄) ₂ or other electron donating compounds containing or able to generate either Fe²⁺or Ti³⁺ irons.

The present invention of option (A) is generally directed to a hydrogen peroxide disinfection solution. The present invention is partly based on the discovery that the addition of a copolymer of vinylpyrrolidone as defined herein to the hydrogen peroxide disinfection solution can provide an increased residual hydrogen peroxide concentration by at least 20%, preferred by at least 35%, or more preferred by at least 50% relative to a control solution having identical composition without the copolymer of vinylpyrrolidone in the presence an identical catalyst. The hydrogen peroxide disinfection solution also contains one or more buffering agents in an amount sufficient to provide the composition a pH of from 6.0 to 8.0, wherein the composition has an osmolality of from 200 to 450 mOsm/kg and a viscosity up to 5.0 centipoises at 25°C following neutralization. The present invention of option (A) is also partly based on the discovery that although the addition of a copolymer of vinylpyrrolidone can provide an substantially increased residual hydrogen peroxide at 30 minutes treatment, the concentration of residual hydrogen peroxide after 6 hours treatment is still reduced to less than 100 ppm, and the contact lens treated with the hydrogen peroxide solution having copolymer of vinylpyrrolidone could still be comfortably inserted to eye. The present invention of option (A) is further partly based on the discovery that the presence of a copolymer of vinylpyrrolidone in the hydrogen peroxide disinfection solution causes no or substantially low foaming when the copolymer of vinylpyrrolidone containing hydrogen peroxide disinfection solution is used to disinfect the contact lens in a catalyst disc containing container. The present invention of option (A) is still further partly based on the discovery that the presence of a copolymer of vinylpyrrolidone in the hydrogen peroxide disinfection solution provides lubricity to the contact lenses, which provides unexpected benefits of increased wettability, reduced friction, initial comfort, and/or reduced adherence of deposits onto the lens, thereby increasing the comfort of the contact lens in the eye.

Although the inventors do not wish to be bound by any particular theory, it is believed that a copolymer of vinylpyrrolidone as defined herein can have an effect on the initial comfort (at the time of inserting the lens). The copolymer of vinylpyrrolidone with sufficiently larger molecular weight can provide lubrication between the lens and the epithelium of the cornea. Incorporation of copolymer of vinylpyrrolidone can increase the lubricity, wettability (characterized by decreased contact angle) and/or reduced friction of the contact lens immersed in the contact lens solution of the present invention.

In accordance with the invention, a contact lens can be a conventional hydrogel contact lens (i.e., a non-silicone hydrogel lens) or preferably a silicone hydrogel contact lens.

The solution of the invention contains hydrogen peroxide in a concentration that is suitable for disinfecting purposes, which is 2% to 6% by weight, most preferably between 3% and 4%, or 3% by weight.

In accordance with the invention, a copolymer of vinylpyrrolidone and at least one amino-containing vinylic monomer is used wherein the amino-containing vinylic monomers are selected from_alkylaminoalkylmethacrylate having 8-15 carbon atoms, alkylaminoalkylacrylate having 7-15 carbon atoms, dialkylaminoalkylmethacrylate having 8-20 carbon atoms, dialkylaminoalkylacrylate having 7-20 carbon atoms, and N-vinylalkylamide having 3-10 carbon atoms. Examples of preferred N-vinyl alkylamide include without limitation N-vinyl formaide, N-vinyl acetamide, N-vinyl isopropylamide, and N-vinyl-N-methyl acetamide.
Examples of preferred copolymers includes without limitation copolymers of vinylpyrrolidone and dimethylaminoethylmethacrylate. Such preferred copolymers are commercially available, e.g., Copolymer 845 and Copolymer 937 from ISP.

The copolymers of vinylpyrrolidone are present in the composition in an amount of from 0.1 % to 5% by weight, preferably 0.1 to 3%; more preferably from 0.5% to 2%, most preferably from 0.25% to 1.5%, based on weight per volume (w/v).
The composition of the present invention preferably contains a buffer. The buffer maintains the pH preferably in the desired range, for example, in a physiologically acceptable range of 4 or 5 or 6 to 8 or 9 or 10. In particular, the solution preferably has a pH in the range of 5.5 to 8. The buffer is selected from inorganic or organic bases, preferably basic acetates, phosphates, borates, citrates, nitrates, sulfates, tartrates, lactates, carbonates, bicarbonates and mixtures thereof, more preferably basic phosphates, borates, citrates, tartrates, carbonates, bicarbonates and mixtures thereof. Typically, it is present in an amount of 0.001% to 2%, preferably 0.01% to 1%; most preferably from about 0.05% to about 0.30%, based on weight per volume (w/v).

The buffer component preferably includes one or more phosphate buffers, for example, combinations of monobasic phosphates, dibasic phosphates and the like. Particularly useful phosphate buffers are those selected from phosphate salts of alkali and/or alkaline earth metals. Examples of suitable phosphate buffers include one or more of sodium dibasic phosphate (Na₂HPO₄), sodium monobasic phosphate (NaH₂PO₄), and potassium monobasic phosphate (KH₂PO₄). Another preferable buffer system includes one or more borate buffers.

The solutions according to the invention are preferably formulated in such a way that they are isotonic with the lacrimal fluid. A solution which is isotonic with the lacrimal fluid is generally understood to be a solution whose concentration corresponds to the concentration of a 0.9% sodium chloride solution (308 mOsm/kg). Deviations from this concentration are possible throughout.

The isotonicity with the lacrimal fluid, or even another desired tonicity, may be adjusted by adding organic or inorganic substances which affect the tonicity. Suitable occularly acceptable tonicity agents include, but are not limited to sodium chloride, potassium chloride, glycerol, propylene glycol, polyethylene glycols, polios, mannitols, sorbitol, xylitol and mixtures thereof. Preferably, the majority of the tonicity of the solution is provided by one or more compounds selected from the group consisting of non-halide containing electrolytes (e.g., sodium bicarbonate) and non-electrolytic compounds. The tonicity of the solution is typically adjusted to be in the range from 200 to 450 milliosmol (mOsm), preferably from 250 to 350 mOsm.

The composition of the present invention may contain a surfactant or a mixture of surfactants. Surfactant can be virtually any acceptable ocular surfactant including non-ionic, anionic, and amphoteric surfactants. Suitable surfactants can be generally described as block copolymers of a hydrophile and hydrophobe terminated in either primary or secondary hydroxyl groups. A first example of such surfactants is polyoxyethylene/polyoxypropylene condensation polymers terminated in primary hydroxyl groups. They may be synthesized by first creating a hydrophobe of desired molecular weight by the controlled addition of propylene oxide to the two hydroxyl groups of propylene glycol. In the second step of the synthesis, ethylene oxide is added to sandwich this hydrophobe between hydrophile groups. Such block copolymers can be obtained commercially from the BASF Corporation under the trademark PLURONIC®. A second example of such surfactants is polyoxyethylene/ polyoxypropylene condensation polymers terminated in secondary hydroxyl groups. They may be synthesized by first creating a hydrophile (polyoxyethylene) of desired molecular weight by the controlled addition of ethylene oxide to ethylene glycol. In the second step of the synthesis, propylene oxide is added to create hydrophobic blocks on the outside of the molecule. Such block copolymers can be obtained commercially from the BASF Corporation under the trademark PLURONIC® R. Specific examples of PLURONIC® R surfactants that are satisfactory include: PLURONIC® 31 R1, PLURONIC® 31 R2, PLURONIC® 25R1, PLURONIC® 17R1, PLURONIC® 17R2, PLURONIC® 12R3. Particularly good results are obtained with PLURONIC® 17R4 surfactant.

The PLURONIC® letter-number combinations are used to identify the various products of the series. The alphabetical designation explains the physical form of the product: 'L' for liquids, 'P' for pastes, 'F' for solid forms (all at 20°C). The first digit (two digits in a three-digit number) in the numerical designation, multiplied by 300, indicates the approximate molecular weight of the hydrophobe (polypropylene oxide). The last digit, when multiplied by 10, indicates the approximate polyethylene oxide content in the molecule in percent.

The letter 'R' found in the middle of the designation of the PLURONIC® R series signifies that this product has a reverse structure compared to the PLURONIC® products, i.e., the hydrophile (ethylene oxide) is sandwiched between the propylene oxide blocks. The numeric designation preceding the 'R', when multiplied by 100, indicates the approximate molecular weight of the propylene oxide block. The number following the 'R', when multiplied by 10, indicates the approximate weight percent ethylene oxide in that product.

Examples of preferred surfactants include without limitation poloxamers (e.g., Pluronic® L35, L43, L44, L62, L62D, L62LF, L64, L92, F108, F123, F88, F98, F68, F68LF, F127, F87, F77, P84 , P85, P75, P103, P104, P105 and 17R4), poloxamines (e.g., Tetronic® 707, 1107 and 1307, polyethylene glycol esters of fatty acids (e.g., Tween® 20, Tween® 80), polyoxyethylene or polyoxypropylene ethers of C₁₂ -C₁₈ alkanes (e.g., Brij@ 35), polyoxyethyene stearate (Myrj® 52), polyoxyethylene propylene glycol stearate (Atlas® G 2612), and amphoteric surfactants under the trade names Mirataine® and Miranol®.

The amount of surfactant component varies over a wide range depending on a number of factors, for example, the specific surfactant or surfactants being used, the other components in the composition and the like. For example, for PLURONIC® series surfactant, often the amount of surfactant is in the range of 0.0001% or 0.0002% to 0.03% or 0.05% or 0.08% (w/v). Preferably, the surfactant is present in an amount less than 0.02%; and most preferably less than 0.04%. Another example, non PLURONIC® R series surfactant, for example, PLURONIC® R often the amount of surfactant is in the range of 0.005% or 0.01% to 0.1% or 0.5% or 0.8% (w/v). Preferably, the surfactant is present in an amount less than 0.2%; and most preferably less than 0.1 %.

A contact lens solution of the invention can include a viscosity-enhancing polymer, which can be a water soluble cellulose-derived polymer, a watersoluble polyvinylalcohol (PVA), or combination thereof. Examples of useful cellulose-derived polymers include without limitation cellulose ethers. Exemplary preferred cellulose ethers are methyl cellulose (MC), ethyl cellulose (EC), hydroxymethylcellulose (HMC), hydroxyethyl cellulose (HEC), hydroxypropylcellulose (HPC), hydroxypropylmethyl cellulose (HPMC), or a mixture thereof. More preferably, cellulose ether is hydroxyethyl cellulose (HEC), hydroxypropylmethyl cellulose (HPMC), and mixtures thereof. The cellulose ether is present in the composition in an amount of preferably from 0.001 % to 0.5% by weight, based on the total amount of the lens care. The desired viscosity of the lens care solution is up to 5.0 centipoises at 25°C.

In accordance with the invention, the solution can further comprises mucin-like materials, ophthalmically beneficial materials, hyaluronic acid and/or surfactants.

Exemplary mucin-like materials include without limitation polyglycolic acid and polylactides. A mucin-like material can be used as guest materials which can be released continuously and slowly over extended period of time to the ocular surface of the eye for treating dry eye syndrome. The mucin-like material preferably is present in effective amounts.

Exemplary ophthalmically beneficial materials include without limitation 2-pyrrolidone-5-carboxylic acid (PCA), amino acids (e.g., taurine, glycine, etc.), alpha hydroxyl acids (e.g., glycolic, lactic, malic, tartaric, mandelic and citric acids and salts thereof, etc.), linoleic and gamma linoleic acids, and vitamins (e.g., B5, A, B6, etc.).

A lens can be prepared according to any methods known to a person skilled in the art from a hydrogel lens-forming formulation. A "hydrogel lens-forming formulation" or "hydrogel lens-forming material" refers to a polymerizable composition which can be cured (i.e., polymerized and/or crosslinked) thermally or actinically to obtain a crosslinked/polymerized polymeric material. Lens-forming materials are well known to a person skilled in the art. Typically a lens forming material comprises polymerizable/crosslinkable components, for example, such as, monomers, macromers, prepolymers, or combinations thereof, as known to a person skilled in the art. A lens-forming material can further include other components, such as non-crosslinkable hydrophilic polymers (i.e., leachable polymeric lubricants), an initiator (e.g., a photoinitiator or a thermal initiator), a visibility tinting agent, UV-blocking agent, photosensitizers, antimicrobial agents, and the like.

The present invention of option (B) provides an increased residual hydrogen peroxide concentration by reducing the surface area of the disc relative to a control trigon disc. The current marketed trigon catalyst disc comprises a generally hollow, and circular, cogwheel-shaped body having a surface area approximately 10.4cm². The catalyst disc is shaped to cooperatively fit within the bottom portion of a cylindrical container. Such cylindrical containers are known in the art, and examples are provided in U.S. 5,196,174. The surface area of catalyst disc can be reduced by reducing the height of the disc or removing one or several arms from the disc. The catalyst discs with surface areas of 10.4 cm² (a trigon disc as a control), 8.7 cm² (a radial disc) and 5.2 cm² (a radial disc) are evaluated as examples. Please note the surface area of the catalyst disc is important for increasing residual hydrogen peroxide concentration after certain treatment time at a given treatment conditions (hydrogen peroxide solution composition, initial hydrogen peroxide concentration, volume of hydrogen peroxide, treatment temperature, etc.).

The present invention of option (B) is generally directed to a hydrogen peroxide catalyst method. The present invention is partly based on the discovery that a smaller catalyst disc can provide an increased residual hydrogen peroxide concentration by at least 80%, preferred by at least 100%, more preferred by at least 500%, still further preferred by 1000% relative to the control disc having a substrate surface area of 10.4 cm² and a substantially identical catalyst coating weight per surface area when in contact with a fixed volume of hydrogen peroxide solution in the container, wherein the radial disc is positioned in the container to be in contact with the aqueous hydrogen peroxide solution. Residual hydrogen peroxide concentration is measured when a fixed volume of hydrogen peroxide contacts with a catalyst coated disc having a less surface area than the control disc in a plastic container for 60 minutes at room temperature. The present invention is also partly based on the discovery that although the smaller catalyst disc can provide an substantially increased residual hydrogen peroxide concentration (80% to 1000%) measured when a fixed volume of hydrogen peroxide contacts with a catalyst coated disc having a less surface area than the control disc in a plastic container for 60 minutes at room temperature, the concentration of residual hydrogen peroxide after 6 hours contacting is reduced to less than 200 ppm, more preferred 100 ppm, and the contact lens treated with the hydrogen peroxide catalyst apparatus could be comfortably inserted to eye.

The present invention provides improved neutralization kinetics by reducing the surface area of the disc. The current marketed trigon disc has a surface area approximately 10.4 cm² with 1150 µg platinum coating, as shown in table 1. The Table 1 also provides a radial disc having a surface area of 8.7 cm² with 968 µg platinum coating; another radial disc having a surface area of 5.2 cm² with 649 µg platinum coating. Surprisingly, a smaller platinum coated disc delivers an increased concentration of hydrogen peroxide to the contact lenses during the first hour of treatment and throughout the cleaning cycle, thereby resulting in an overall increase in disinfection efficacy. This assumes the hydrogen peroxide disinfecting solution used to disinfect a contact lens has a volume in the range of 8 ml to 18 ml. In the preferred embodiment, the hydrogen peroxide disinfecting solution has a volume in the range of 9.5 ml to 11.5 ml. In the preferred embodiment, the radial disc has a surface area in the ranges of 2.0 cm² to 9.0 cm², preferred surface area in the range from 3.5 cm² to 8.5 cm², more preferred surface area in the range from 5.0 cm² to 6.5 cm², furthermore preferred surface area in the range from 5.2 cm² to 6.0 cm².

In the preferred embodiment, the radial disc employs a plastic carrier and has a catalyst coating in the ranges of 200 µg to 1000 µg, preferred a catalyst coating in the range from 400 µg to 960 µg, more preferred a catalyst coating in the range from 500 µg to 700 µg, furthermore preferred catalyst coating in the range from 600 µg to 650 µg.

From the point of view of coating weight per surface area, in the preferred embodiment, the disc has a catalyst coating weight per surface area which is from 60 µg / cm² to 250 µg / cm², preferred from 90 µg / cm² to 135 µg / cm², more preferred from 100 µg / cm² to 125 µg / cm².

After six hours, the residual peroxide concentration is still maintained below levels that may result in burning/stinging and/or corneal irritation.

**Table 1**

| | | **Residual H202 concentration** | |
|---|---|---|---|
| | **Surface Area** | **Actual Measured Concentration** | |
| **Currently marketed trigon disc** | 10.4 cm² | 1 hour | 6 hours |
| | | 177 ppm | 1.93 ppm |
| **New (surface-area modified) radial disc** | 8.7 cm² | 360 ppm | 4.8 ppm |
| **50% Height-modified radial disc** | 5.2 cm² | 1800 ppm | 25 pm |

Further, the present invention provides for a catalyst covered radial disc using a relatively simple and straightforward coating composition which provides for ease in manufacturing the present compositions and decreases the number of potential problems which may result using such compositions. The methods by which the compositions are produced provide coatings which are highly uniform and, therefore, highly reliable in providing the desired hydrogen peroxide degradation kinetics.

The present invention of option (B) is directed to a hydrogen peroxide catalyst kit to effect the neutralization of an aqueous hydrogen peroxide disinfecting solution that provides disinfection of contact lens and render it suitable for insertion into the eye upon completion of disinfection. The kit may comprise a radial disc having a surface area of approximately 2.0 cm² to 9.0 cm². The radial disc may further comprise a catalyst coating of 200 µg to 900 µg that delivers increased efficacy in disinfecting the contact lenses during the first hour of treatment. In the preferred embodiment, the radial disc has a surface area in the ranges of from 3.5 cm² to 8.5 cm², more preferred surface area in the range from 5.0 cm² to 6.5 cm², furthermore preferred surface area in the range from 5.2 cm² to 6.0 cm². In the preferred embodiment, the radial disc has a catalyst coating in the ranges of from 400 µg to 960 µg, more preferred a catalyst coating in the range from 500 µg to 700 µg, furthermore preferred catalyst coating in the range from 600 µg to 650 µg. In the still further preferred embodiment, the disc has a catalyst coating weight per surface area which is from 60 µg / cm² to 250 µg / cm², preferred from 90 µg / cm² to 135 µg / cm², more preferred from 100 µg / cm² to 125 µg / cm².

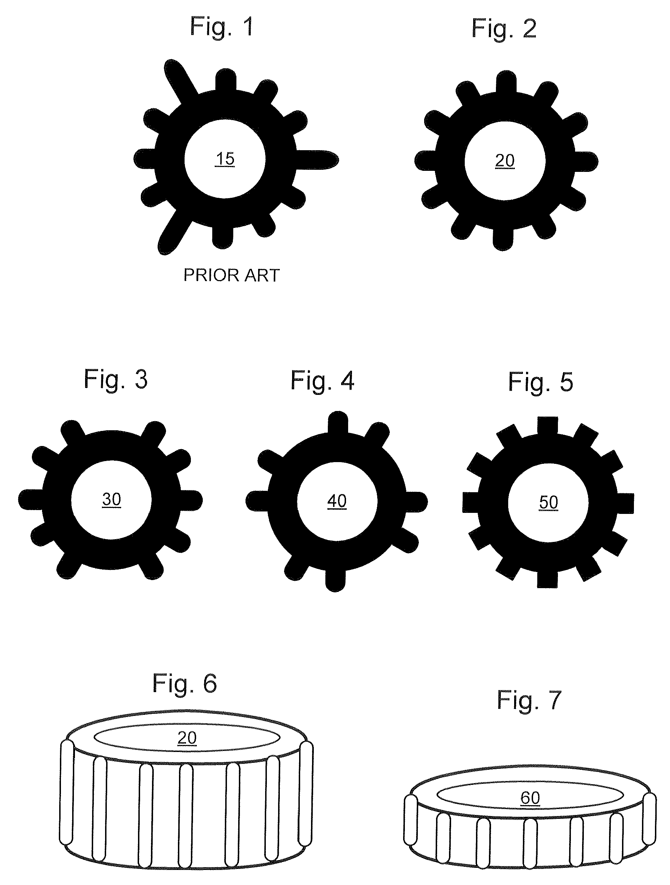

With reference now to the figures, like reference numbers represent corresponding parts throughout the several views. Figure 1 shows a top view of a Trigon disc catalyst disc 15 having a surface area of 10.4 cm² of the prior art. The Trigon catalyst disc comprises a generally hollow, and circular, cogwheel-shaped body with three longer arms than the rest of arms. The catalyst disc is shaped to have a central hollow hole to cooperatively fit within the bottom portion of a cylindrical container. Such cylindrical containers are known in the art, and examples are provided in U.S. 5,196,174.

Figure 2 shows a top view of a radial catalyst disc control 20 having a surface area of 8.74 cm² according to an example embodiment of the present invention. As illustrated, the radial disc 20 extends arms radically from the core. As shown the radial disc control 20 has a similar generally hollow, circular, cogwheel- shape but a reduced surface area from the trigon disc of the prior art by making the all arms of the disc with equal length. This reduced surface area of the radial disc provides for a slower reaction with the hydrogen peroxide, thereby maintaining higher levels of hydrogen peroxide early in the cleaning process while still reducing the residual hydrogen peroxide to below levels that may result in burning/stinging and/or cornea irritation. A perspective view of the radial catalyst disc is shown in Figure 6.

Figure 3-5 are top views of a radial catalyst disc having a similar generally hollow, circular, cogwheel- shape but a smaller surface area than the radial disc catalyst of the prior art with various modifications to reduce the surface area. In Figure 3, the radial catalyst disc 30 having a surface area of 7.79 cm² is shown with 2 arms shaved off. In Figure 4, the radial catalyst disc 40 is shown with 4 arm shaved off and have a surface area of 6.80 cm². In Figure 5, the radial catalyst disc 50 is shown with shaved arms that are squared off on the tips. These various designs around a similar core item to allow for progressive effectiveness of the radial disc of the present invention. In this way the appropriate radial disc configuration can be suggested for each different type of hydrogen peroxide disinfecting solution.

In an alternative embodiment, a reduced height radial catalyst disc 60 provides the greatest reduction of surface area. The height of the radial disc 60 is reduced to a size in the range of 5% to 90% of the height of the radial disc 20. This results in a surface area of 1.967 cm² to 8.026 cm². A reduction of approximately 50% of the height of the radial disc is shown in Figure 7, and these results in a surface area of 5.17 cm². This reduction has an enormous impact on the radial disc's peroxide degradation kinetics during the neutralization of a hydrogen peroxide cleaning solution.

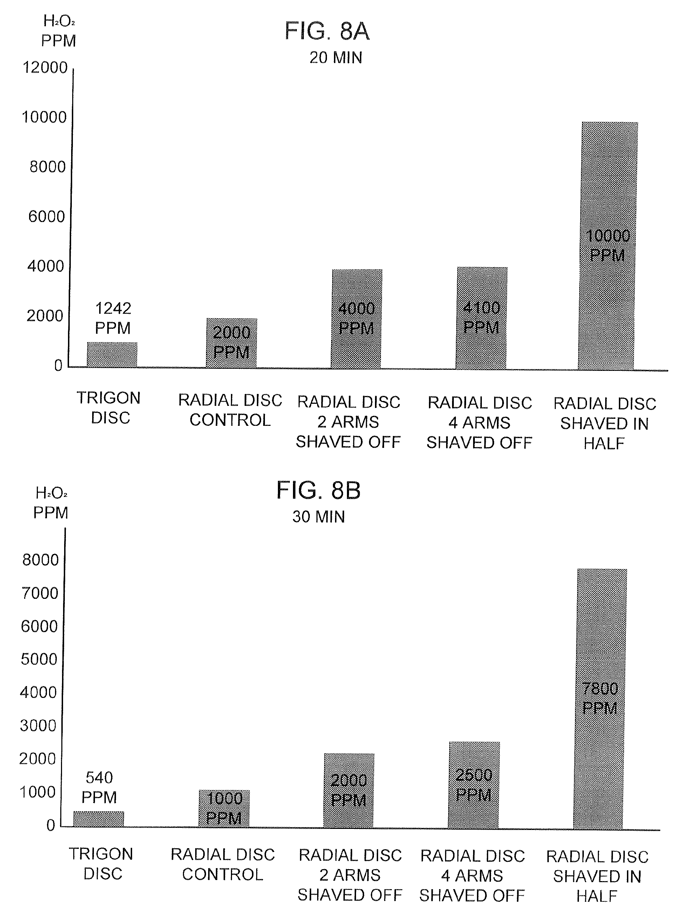

The comparisons of the above described radial discs and their residual hydrogen peroxide concentrations after cycling are illustrated in Figures 8A-8E. The residual hydrogen peroxide concentrations for each of the surface area modified radial catalyst discs 20-40 and 60 soaking in a solution of Clear Care™ is shown for time increments of 20, 30, 60, 120 and 360 minutes. The hydrogen peroxide concentration in Clear Care™ solution is initially about 35,000 ppm.

As shown in Figure 8A, the initial hydrogen peroxide concentration has undergone rapid catalytic neutralization after approximately 20 minutes. After 20 minutes, the radial disc 20 resulted in neutralization of the hydrogen peroxide to approximately 2000 ppm. This is in contrast to the reduced catalytic neutralization for the radial disc 30, 40 and 60. The two arms reduced radial disc 30 results in approximate hydrogen peroxide concentrations of 4000 ppm, the four arms reduced radial disc 40 results in hydrogen peroxide concentrations of approximately 4100 ppm and the 50% height modified radial disc 60 results in hydrogen peroxide concentrations of approximately 10,000 ppm. Figure 8B illustrates the hydrogen peroxide concentrations after cycling 30 minutes using the 20-40 and 60 radial discs, the measurements were approximately 1000 ppm, 2000 ppm, 2500 ppm, and 7800 ppm respectively for each of the radial discs.

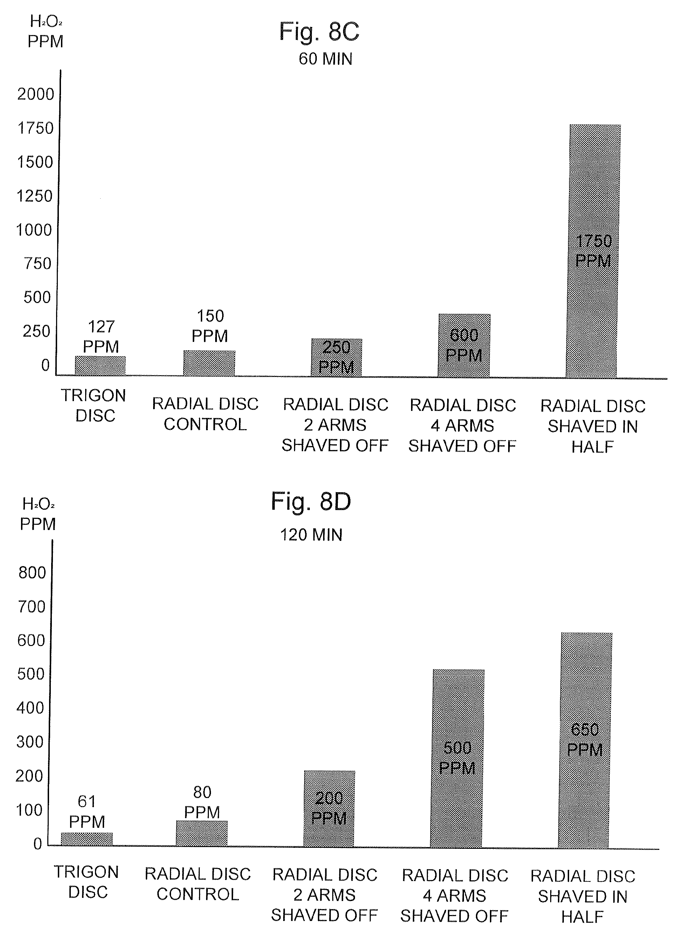

Figure 8C illustrates the measured hydrogen peroxide concentrations after cycling approximately 60 minutes for each of the radial discs 20-40 and 60. The hydrogen peroxide concentrations for each of the radial discs 20-40 and 60 were approximately 150, 250, 600 and 1750 ppm respectively. As can be seen, the reduction in surface area of the radial discs has a magnified impact on the neutralization of the hydrogen peroxide in the disinfecting solution. The lower the surface area of the catalyst coated disc, the slower the neutralization.

Figure 8D illustrates the hydrogen peroxide concentrations after cycling 120 minutes using the 20-40 and 60 radial discs, the measurements were approximately 80 ppm, 200 ppm, 500 ppm, and 650 ppm respectively for each of the radial discs.

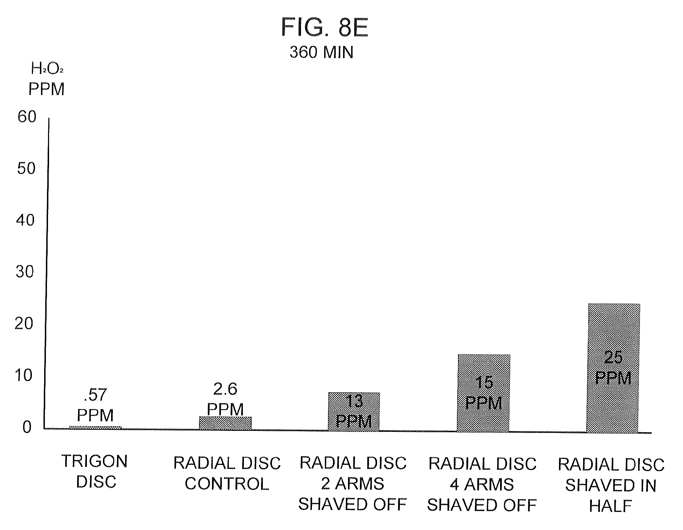

Figure 8E illustrates the hydrogen peroxide concentrations after cycling 6 hours using the 20-40 and 60 radial discs, the measurements were approximately 2.6 ppm, 13 ppm, 15 ppm, and 25 ppm respectively for each of the radial discs. The use of radial disc 20-40 and 60 should not increase the risk of burning and stinging associated with a high residual peroxide content because the residual hydrogen peroxide concentrations are below 100 ppm after 6 cycling hours.

From the above, it is readily apparent that the rate of neutralization of the hydrogen peroxide is dependent upon catalyst area provided. Accordingly, any desired rate of hydrogen peroxide neutralization may be achieved by increasing or decreasing the surface area of the catalyst coated radial disc employed.

The present invention of option (B) is generally directed to provide a kit for use in the disinfection of contact lenses comprising: a) an aqueous hydrogen peroxide solution as disclosed in claim 1, and b) a container for holding a given amount of hydrogen peroxide solution, wherein the container contains a disc wherein the disc comprises a substrate and a catalyst coating thereon, wherein, when the given amount of hydrogen peroxide is added to the container to entirely submerge the catalyst coated radial disc, the aqueous hydrogen peroxide solution decomposes over a period time of six hours to provide a residual hydrogen peroxide concentration of less than 100 ppm.

The previous disclosure will enable one having ordinary skill in the art to practice the invention. In order to better enable the reader to understand specific embodiments and the advantages thereof, reference to the following non-limiting examples is suggested. However, the following examples should not be read to limit the scope of the invention.

The following non-limiting examples illustrate certain aspects of the present invention.

Example 1 (PRIOR ART) -Clear Care solution is commercially available from Ciba Vision.

**A quantity of the following liquid composition is prepared by blending together the individual ingredients (in % by weight per volume (w/v)).**

| | |
|---|---|
| Hydrogen peroxide | 3.5% |
| Sodium Phosphate, Monobasic monohydrate | 0.072% |
| Sodium Phosphate, Dibasic anhydrous | 0.1555% |
| DEQUEST® 2060S | 0.012% |
| Sodium Chloride | 0.79% |
| PLURONIC 17R4 | 0.05% |
| Purified Water | q.s. to volume |

The resulting solution is an aqueous solution containing 3.5% hydrogen peroxide; 0.072% sodium phosphate, monobasic monohydrate; 0.1555% sodium phosphate, dibasic anhydrous; 0.012% DEQUEST® 2060S; and 0.79% sodium chloride and 0.05% PLURONIC 17R4.

### Example 2

A solution was prepared in the same manner as Example 1, except 1% COPOLYMER 845 was added to the solution. COPOLYMER 845 was obtained from ISP.

### Example 3

A solution was prepared in the same manner as Example 1, except 1% COPOLYMER 845 and 0.02% HPMC E4M were added to the solution. COPOLYMER 845 was obtained from ISP, and HPMC E4M was obtained from DOW.

### Example 4

A solution was prepared in the same manner as Example 1, except 1.0% COPOLYMER 845 was added to the solution. PLURONIC P103 (0.001 %) was substituted for the PLURONIC 17R4. PLURONIC P103 was obtained from BASF.

### Example 5

A solution was prepared in the same manner as Example 1, except 0.5% COPOLYMER 845 was added to the solution. PLURONIC P103 at 0.001% was substituted for the PLURONIC 17R4.

### Example 6

A solution was prepared in the same manner as Example 1, except the phosphate buffer was substituted with 0.64% borate buffer, and 1.0% COPOLYMER 845 and 0.02% HPMC E4M were added to the solution. Peroxide concentration after 30 minutes and 360 minutes peroxide neutralization
(using the AOCUP and AODisc platinum coated disc system; both are commercially available as sold with AOSEP® hydrogen peroxide system)

**Table 1A - Peroxide content following disinfection at a specific time point (30 minutes or 360 minutes) in the AOCup System with AODisc for the above mentioned Examples**

| Contact lens solution | 30 Minutes Neutralization | 360 Minutes Neutralization |
|---|---|---|
| Example 1 (n=5) | 1080.64 ppm +/- 99.52 ppm | 2.75 ppm +./- 0.07 ppm |
| Example 2 (n=4) | 1678.33 ppm +/- 124.92 ppm | 8.05 ppm +/- 0.21 ppm |
| Example 3 (n=15) | 2397.11 ppm +/- 297.26 ppm | 12.60 ppm +/- 0.28 ppm |
| Example 4 (n=6) P26 | 2055.56 ppm +/- 186.94 ppm | 9.15 ppm +/- 0.56 ppm |
| Example 5 (n=6) P35 | 2385.00 ppm +/- 606.21 ppm | 14.78 ppm +/- 1.18 ppm |
| Example 6 (n=6) N35 | 2063.33 ppm +/- 185.53 ppm | 12.85 ppm +/- 0.54 ppm |

**Table 1B - Peroxide content following disinfection at a specific time point (60 minutes in the AOCup System with AODisc for the above mentioned Examples I. II and III**

| Contact lens solution | 60 Minutes Neutralization |
|---|---|
| Example 1 (n=4) | 499.5 ppm +/- 29.86 ppm |
| Example 2 (n=2) | 826 ppm +/- 16.97 ppm |
| Example 3 (n=4) | 824.0 ppm +/- 22.33 ppm |

### Examples 7-9

A solution used in examples 7-9 was prepared in the same manner as Example 1 (prior art). The hydrogen peroxide - decomposition radial disc has a substrate to have a surface area of 10.4 cm², 8.7 cm² and 5.2 cm², respectively.

### Examples 10-12

A solution used in examples 10-12 was prepared in the same manner as Example 3. The hydrogen peroxide - decomposition radial disc has a substrate to have a surface area of 10.4 cm², 8.7 cm² and 5.2 cm², respectively.

**Table 2- Peroxide content following disinfection at a specific time point (360 minutes) in the AOCup System with Disc with specified surface area for the above mentioned Examples**

| | Sample Description | 6 hr residual H202 conc.(ppm) | Average conc. | Std deviation |
|---|---|---|---|---|
| Example 7 | Trigon disc with surface of 10.4 cm² | 6.6 | 5.33 | 1.21 |
| | | 5.2 | | |
| | | 4.2 | | |
| Example 8 | Radial disc with surface of 8.7 cm² | 14.4 | 13.00 | 1.98 |
| | | 11.6 | | |
| Example 9 | Radial disc with surface of 5.2 cm² | 32.5 | 37.00 | 6.36 |
| | | 41.5 | | |
| Example 10 | Trigon disc with surface of 10.4 cm² | 29.4 | 26.10 | 4.67 |
| | | 22.8 | | |
| Example 11 | Radial disc with surface of 8.7 cm² | 35 | 36.5 | 2.12 |
| | | 38 | | |
| Example 12 | Radial disc with surface of 5.2 cm² | 58.5 | 53.75 | 6.72 |
| | | 49 | | |

The combination of the radial disc with a surface area of 10.4 cm² and 8.7 cm² with the solution 3 (the hydrogen peroxide solution having copolymer of vinylpyrrolidone) results in a residual hydrogen peroxide concentrations that are slightly above the 20 ppm, with 26 and 36 ppm. However, the use of radial discs with a surface area of 10.4 cm² and 8.7 cm² with the solution 3 with the solution 3 should not significantly increase the risk of burning and stinging associated with a high residual peroxide content.

Radial disc with a surface of 5.2 cm² performed in the solution 1 (Clear Care™ solution) nearly as well as the radial disc with a surface area of 8.7 cm² in the solution 3 solution (the hydrogen peroxide solution having copolymer of vinylpyrrolidone). After six hours, the residual hydrogen peroxide concentration is approximately 37 ppm. This is well less than 100 ppm and the contact lens treated with the hydrogen peroxide solution having copolymer of vinylpyrrolidone could still be comfortably inserted to eye. The radial disc with surface of 5.2 cm² in the Example 3 solution had the highest residual hydrogen peroxide concentration of all the combinations tested. The residual hydrogen peroxide concentration for the Radial disc with surface of 5.2 cm² in the Example 3 solution is approximately 54 ppm. This is still well less than 100 ppm and the contact lens treated with the hydrogen peroxide solution having copolymer of vinylpyrrolidone could still be comfortably inserted to eye.

## Claims

1. A solution for disinfecting a contact lens comprising an effective disinfecting amount of hydrogen peroxide of 2% to 6% by weight and a copolymer of vinylpyrrolidone, wherein the copolymer of vinylpyrrolidone is present in an amount of 0.1 % to 5% by weight per volume (w/v) sufficient to provide an increased residual hydrogen peroxide concentration at 30 minutes treatment by at least 20% relative to a control solution having identical composition without the copolymer of vinylpyrrolidone in the presence of an identical catalyst, one or more buffering agents in an amount sufficient to provide the composition a pH of from 6.0 to 8.0, wherein the composition has an osmolality of from 200 to 450 mOsm/kg and a viscosity up to 5.0 centipoises at 25°C, wherein the copolymer is a copolymer of vinylpyrrolidone and at least one amino-containing vinylic monomer, wherein the amino-containing vinylic monomer is selected from the group consisting of alkylaminoalkylmethacrylate having 8-15 carbon atoms, alkylaminoalkylacrylate having 7-15 carbon atoms, dialkylaminoalkyl-methacrylate having 8-20 carbon atoms, dialkylaminoalkylacrylate having 7-20 carbon atoms, and N-vinylalkylamide having 3-10 carbon atoms.

2. The solution of claim 1, wherein the amino-containing vinylic monomer is dimethylaminoethylmethacrylate or dimethylaminoethylacrylate.

3. The solution of claims 1-2 further comprising a viscosity builder selected from a group consisting of methyl cellulose (MC), ethyl cellulose, hydroxymethylcellulose, hydroxyethyl cellulose (HEC), hydroxypropylcellulose (HPC), hydroxypropylmethyl cellulose (HPMC) and mixtures thereof.

4. The solution of claims 1-3 further comprising a buffering agent selected from a group consisting of phosphate buffers and borate buffer.

5. The solution of claims 1-4 further comprising a surfactant selected from a group consisting of Pluronic 17R4 and Pluronic P103.

6. The solution of claims 1-5, wherein the concentration of peroxide is 3% based on the total weight of the solution.

7. The solution of claim 1, wherein the concentration of the copolymer of vinylpyrrolidone is 0.25% to 1.5% by weight per volume (w/v).

8. The solution of claims 1-7, wherein the increased residual hydrogen peroxide concentration is at least 35%

9. The solution of claim 8, wherein the increased residual hydrogen peroxide concentration is at least 50%

10. A kit for use in the disinfection of contact lenses comprising:
a) an aqueous hydrogen peroxide solution of claims 1-9, and b) a container for holding a given amount of hydrogen peroxide solution, wherein the container contains a disc, wherein the disc comprises a substrate and a catalyst coating thereon, wherein, when the given amount of hydrogen peroxide is added to the container to entirely submerge the catalyst coated disc, the aqueous hydrogen peroxide solution decomposes over a period time of six hours to provide a residual hydrogen peroxide concentration of less than 100 ppm.

11. The kit of claim 10, wherein the disc is a trigon disc with a surface area of 10.4cm².

12. The kit of claim 10, wherein the disc is a radial disc with a surface area of 2.0 cm² to 9.0 cm².

## Patentansprüche

1. Eine Lösung zur Desinfektion von Kontaktlinsen enthaltend eine wirksam desinfizierende Menge Wasserstoffperoxid von 2 bis 6 Gew.-% und ein Vinylpyrrolidoncopolymer, wobei das Vinylpyrrolidoncopolymer in einer Menge von 0,1 bis 5 % Gewicht auf Volumen (w/v) anwesend ist, die ausreicht, eine bei 30-minütiger Behandlung zumindest um 20% erhöhte Restwasserstoffperoxidkonzentration zu liefern, gegenüber einer Kontrolllösung identischer Zusammensetzung ohne Vinylpyrrolidon-copolymer in Gegenwart eines identischen Katalysators, ein oder mehrere Pufferungsmittel in einer Menge, die ausreicht, um der Zusammensetzung einen pH von 6,0 bis 8,0 zu verleihen, wobei die Zusammensetzung eine Osmolalität von 200 bis 450 mOsm/kg und eine Viskosität von bis zu 5,0 Centipoise bei 25°C hat, wobei das Copolymer ein Copolymer von Vinylpyrrolidon und zumindest einem amingruppen-haltigen vinylischen Monomer ist, wobei das vinylische amingruppen-haltige Monomer ausgewählt ist aus der Gruppe bestehend aus Alkylaminoalkylmethacrylat enthaltend 8-15 Kohlenstoffatome, Alkylaminoalkylacrylat enthaltend 7-15 Kohlenstoffatome, Dialkylaminoalkylmethacrylat enthaltend 8-20 Kohlenstoffatome, Dialkylaminoalkylacrylat enthaltend 7-20 Kohlenstoffatome, und N-Vinylalkylamid enthaltend 3-10 Kohlenstoffatome.

2. Die Lösung des Anspruchs 1, wobei das amingruppenhaltige vinylische Monomer Dimethylaminoethylmethacrylat oder Dimethylaminoethylacrylat ist.

3. Die Lösung der Ansprüche 1-2, enthaltend weiterhin einen Viskositätsbildner ausgewählt aus der Gruppe bestehend aus Methylzellulose (MC), Ethylzellulose, Hydroxymethylzellulose, Hydroxyethylzellulose (HEC), Hydroxypropylzellulose (HPC) Hydroxypropylmethylzellulose (HPMC) und Gemischen davon.

4. Die Lösung der Ansprüche 1-3, enthaltend weiterhin ein Pufferungsmittel ausgewählt aus der Gruppe bestehend aus Phosphatpuffern und Boratpuffern.

5. Die Lösung der Ansprüche 1-4, enthaltend weiterhin eine grenzflächenaktive Substanz ausgewählt aus der Gruppe bestehend aus Pluronic 17R4 und Pluronic P103.

6. Die Lösung der Ansprüche 1-5, wobei die Konzentration an Peroxid 3% bezogen auf das Gesamtgewicht der Lösung beträgt.

7. Die Lösung des Anspruchs 1, wobei die Konzentration des Vinylpyrrolidoncopolymers 0,25% bis 1,5% Gewicht auf Volumen (w/v) beträgt.

8. Die Lösung der Ansprüche 1-7, wobei die erhöhte Restwasserstoffperoxidkonzentration mindestens 35% beträgt.

9. Die Lösung des Anspruchs 8, wobei die erhöhte Restwasserstoffperoxidkonzentration mindestens 50% beträgt.

10. Ein Kit zur Verwendung in der Desinfektion von Kontaktlinsen enthaltend:
(a) eine wässrige Wasserstoffperoxidlösung der Ansprüche 1-9, und
(b) einen Behälter zur Aufnahme einer vorgegebenen Menge Wasserstoffperoxidlösung, wobei der Behälter eine Scheibe enthält, wobei die Scheibe ein Substrat und darauf eine Katalysatorbeschichtung umfasst, wobei sich, wenn dem Behälter die vorgegebene Menge Wasserstoffperoxid zugegeben wird, um die katalysatorbeschichtete Scheibe vollständing einzutauchen, die wässrige Wasserstoffperoxidlösung während einer Zeitspanne von sechs Stunden zersetzt, um eine restliche Wasserstoffperoxidkonzentration von weniger als 100 ppm zu ergeben.

11. Der Kit von Anspruch 10, wobei die Scheibe eine Trigonscheibe mit einer Oberflächenausdehnung von 10,4 cm² ist.

12. Der Kit von Anspruch 10, wobei die Scheibe eine strahlenförmige Scheibe mit einer Oberflächenausdehnung von 2,0 cm² bis 9,0 cm² ist.

## Revendications

1. Solution servant à désinfecter une lentille de contact, comprenant une quantité désinfectante efficace de peroxyde d'hydrogène de 2% à 6% en poids et un copolymère de vinylpyrrolidone, dans laquelle le copolymère de vinylpyrrolidone est présent en une quantité de 0,1% à 5% en poids par volume (p/v), suffisante pour permettre une augmentation de la concentration de peroxyde d'hydrogène résiduel, au bout de 30 minutes de traitement, d'au moins 20% par rapport à une solution témoin ayant une composition identique sans le copolymère de vinylpyrrolidone en présence d'un catalyseur identique, d'un ou plusieurs agents tampons en quantité suffisante pour que le pH de la composition soit de 6,0 à 8,0, où la composition a une osmolalité de 200 à 450 mOsm/kg et une viscosité allant jusqu'à 5,0 centipoises à 25°C, où le copolymère est un copolymère de vinylpyrrolidone et d'au moins un monomère vinylique aminé, où le monomère vinylique aminé est choisi dans le groupe constitué par du méthacrylate d'alkylaminoalkyle ayant 8-15 atomes de carbone, de l'acrylate d'alkylaminoalkyle ayant 7-15 atomes de carbone, du méthacrylate de dialkylaminoalkyle ayant 8-20 atomes de carbone, de l'acrylate de dialkylaminoalkyle ayant 7-20 atomes de carbone et du N-vinylalkylamide ayant 3-10 atomes de carbone.

2. Solution selon la revendication 1, dans laquelle le monomère vinylique aminé est le méthacrylate de diméthylaminoéthyle ou l'acrylate de diméthylaminoéthyle.

3. Solution selon les revendications 1-2, comprenant en outre un agent pour augmenter la viscosité, choisi dans un groupe constitué par la méthylcellulose (MC), l'éthylcellulose, l'hydroxyméthylcellulose, l'hydroxyéthylcellulose (HEC), l'hydroxypropylcellulose (HPC), l'hydroxypropylméthylcellulose (HPMC) et leurs mélanges.

4. Solution selon les revendications 1-3, comprenant en outre un agent tampon choisi dans un groupe constitué par les tampons phosphates et le tampon borate.

5. Solution selon les revendications 1-4, comprenant en outre un tensioactif choisi dans un groupe constitué par Pluronic 17R4 et Pluronic P103.

6. Solution selon les revendications 1-5, dans laquelle la concentration de peroxyde est de 3%, basée sur le poids total de la solution.

7. Solution selon la revendication 1, dans laquelle la concentration du copolymère de vinylpyrrolidone est de 0,25% à 1,5% en poids par volume (p/v).

8. Solution selon les revendications 1-7, dans laquelle l'augmentation de la concentration de peroxyde d'hydrogène résiduel est d'au moins 35%.

9. Solution selon la revendication 8, dans laquelle l'augmentation de la concentration de peroxyde d'hydrogène résiduel est d'au moins 50%.

10. Kit à utiliser pour désinfecter les lentilles de contact, comprenant :
a) une solution aqueuse de peroxyde d'hydrogène selon les revendications 1-9, et b) un récipient destiné à contenir une quantité donnée de solution de peroxyde d'hydrogène, dans lequel le récipient contient un disque, où le disque comprend un substrat et un revêtement de catalyseur, où, lorsque la quantité donnée de peroxyde d'hydrogène est ajoutée dans le récipient jusqu'à l'immersion complète du disque à revêtement de catalyseur, la solution aqueuse de peroxyde d'hydrogène se décomposé sur une période de temps de six heures pour procurer une concentration de peroxyde d'hydrogène résiduel inférieure à 100 ppm.

11. Kit selon la revendication 10, dans lequel le disque est un disque trigonal ayant une superficie de 10,4 cm².

12. Kit selon la revendication 10, dans lequel le disque est un disque radial ayant une superficie de 2,0 cm² à 9,0 cm².
